(19) Europäisches Patentamt
European Patent Office
Office européen des brevets

(11) Publication number: 0 297 547 A2

# (12) EUROPEAN PATENT APPLICATION

(21) Application number: 88110386.5

(51) Int. Cl.4 A61K 31/70

(22) Date of filing: 29.06.88

The title of the invention has been amended (Guidelines for Examination in the EPO, A-III, 7.3).

(30) Priority: 29.06.87 JP 161572/87

(43) Date of publication of application: 04.01.89 Bulletin 89/01

(84) Designated Contracting States: DE FR GB IT

(71) Applicant: LEAD CHEMICAL COMPANY LTD.
77-3, Mimata Toyama-shi
Toyama-ken(JP)

(72) Inventor: Mori, Masao
248, Tenshoji
Toyama-shi Toyama-ken(JP)
Inventor: Koshiura, Ryozo
2-5-3, Mitsukuchi Shin-machi
Kanazawa-shi Ishikawa-ken(JP)
Inventor: Kurimura, Tadashi
110-1, Uchi-machi
Yonago-shi Tottori-ken(JP)
Inventor: Okuda, Takuo
3-4-25 Kitakata
Okayama-shi Okayama-ken(JP)

(74) Representative: Brauns, Hans-Adolf, Dr. rer. nat. et al
Hoffmann, Eitle & Partner, Patentanwälte
Arabellastrasse 4
D-8000 Munich 81(DE)

## (54) Use of hydrolyzable tannins for treatement and prophylaxis of AIDS.

(57) Disclosed is a medical composition for remedy of acquired immune deficiency syndrome (AIDS) and for prevention of manifestation of the symptoms of the disease, which comprises tannins containing monomers and oligomers of hydrolyzable tannins as an active ingredient. The medical composition can be administered by any route of peroral or parenteral administration. This may have an elevated infection-inhibiting action when used together with an antibody to HIV.

EP 0 297 547 A2

# MEDICAL COMPOSITIONS FOR REMEDY OF ACQUIRED IMMUNE DEFICIENCY SYNDROME (AIDS) AND FOR PREVENTION OF MANIFESTATION OF THE SYMPTOMS OF THE DISEASE

## Background of the Invention:

### 1. Field of the Invention

The present invention relates to a medical composition for remedy of acquired immune deficiency syndrome (AIDS) and for prevention of manifestation of the symptoms of the disease and, more precisely, to that containing compounds of tannins.

### 2. Description of the Prior Art:

AIDS virus (HIV) is a human Retrovirus, which selectively interferes with the growth of T4 lymphocytes to cause manifestation of the symptoms of AIDS.

AIDS is a cellular immune deficiency syndrome which is caused by infection of T4 (helper inducer) lymphocytes with HIV followed by cytoclasis of the lymphocytes by the HIV.

After having invaded into a human body, HIV invades into the helper inducer T cells in the blood. The virus is thus bonded to the receptor as existing on the surface of the T cell. Then, after having invaded into the cytoplasm of the cell, this forms DNA from the autogenome RNA because of the action of the reverse transcriptase thereof. The DNA is taken into the nucleus of the lymphocyte as a provirus.

In the next stage, the provirus is transcribed into mRNA to give an virus protein. The thus formed virus then germinates from the cell membrane and thereafter invades into other lymphocytes. By the invation of the AIDS viruses the number of the helper inducer T cells decreases, whereby the function of the cells lowers to cause, as a result, the immune deficiency.

For remedy and prevention of AIDS various medicines and vaccines are being studied at present, and for example, antiviral agents such as azidethymidine, dideoxycytidine, dideoxyadenosine, $\alpha$-interferon, ammonium-21-tungsto-9-antimoniate (HAP-23), etc. as well as immunoenhancers such as interleukin, isoprinosine, lentinan, etc. have been proposed.

However, the antiviral agents could not completely kill the AIDS virus, although these are admitted to have the viral propagation-inhibitory action, and additionally, the agents have other various problems. For instance, azidethymidine has harmful side effects of decreasing leukocytes or depressing bone marrow. Some employment of the agent would cause viral tolerance. Accordingly, the agent has a defect that this cannot be used for AIDS virus-carriers. In addition, the agent is considered to be a medicine which is expected to have a mere life-prolonging effect for AIDS patients. Regarding the vaccines, since the AIDS viruses greatly and widely mutate, it is difficult to prevent the AIDS disease by the use of a single vaccine. After manifestation of the symptoms of the AIDS disease, the patient becomes to have an immune deficiency, and therefore the remedy of the AIDS disease with medicines becomes extremely difficult. However, the prevention of manifestation of the symptoms of the AIDS disease in an AIDS virus-carrier, if possible, would be extremely helpful for bleeders or AIDS virus-carriers.

Tannins are polyphenol derivatives of some kinds as contained in plants, and these are grouped into two groups comprising hydrolyzable tannins and condensed tannins. Among the former hydrolyzable tannins, those capable of forming ellagic acid by hydrolysis are called ellagitannins.

## Summary of the Invention:

Tannins have been used as a hide-tanning agent for a long time and additionally have also been used as an internal medicine for digestive system diseases. The present inventors have previously noted that some ellagitannins have a function of noticeably inhibiting the mutagenic action of carcinogens of some kinds and have confirmed that ellagitannins could be used as a carcinostatic. We have further earnestly studied various kinds of tannins and, as a result, have found that compounds of tannins have an action of strongly inhibiting the propagation of HIV.

The present invention has been attained on the basis of the said discovery, and the object of the present invention is to provide a medical composition for remedy of acquired immune deficiency syndrome (AIDS) and for prevention of manifestation of the symptoms of the disease.

Specifically, the medical composition for remedy of acquired immune deficiency syndrom (AIDS) and for prevention of manifestation of the symptoms of the disease is characterized by comprising tannins containing monomers and oligomers of hydrolyzable tannins.

The medicine of the present invention is extremely effective for remedy of acquired immune deficiency syndrome (AIDS) and for prevention of manifestation of the symptoms of the disease. It may effectively be administered by various administration routes, for example, peroral (including buccal tablet and sublingual tablet), perrectal, pernasal, peranal, parenteral (including subcutaneous, intramuscular, intravenous, intraarterial and intracutaneous administration) or the like administration routes. In particular, it may especially preferably be administered by intravenous injeciton. The viral infection-inhibitory activity may be elevated by combination of the medicine of the present invention and an antibody to HIV.

## Detailed Description of the Invention:

Regarding ellagic acid which is a hydrolyzed product of all ellagitannins in tannins, it is known that the said acid has an action of inhibiting the mutagenic action of benzo[α]-pyrene-7,8-diol-9,10-epoxide which is a carcinogen. However, it has not been clarified up to the present that the said acid would have an action of inhibiting Retrovirus or an antiviral action. Accordingly, the present inventors are the first who found the anti-AIDS-viral action of the tannins as extracted from plants and confirmed.

As the compounds of tannins which are active components in the medical composition of the present invention for remedy of acquired immune deficiency syndrome (AIDS) and for prevention of manifestation of the symptoms of the disease, at least one compound selected from the group consisting of dimers such as Nobotanin A, Nobotanin C, Gemin A, Rugosin D, Rugosin E, Isorugosin D, Cornusiin A, Coriariin A, Coriariin C, Oenothein B, Agrimoniin, etc., monomers such as Geraniin, Granatin A, Granatin B, etc., and trimers such as Cornusiin C, etc. The structural formula and properties of these compounds are mentioned hereunder.

(1) Nobotanin A

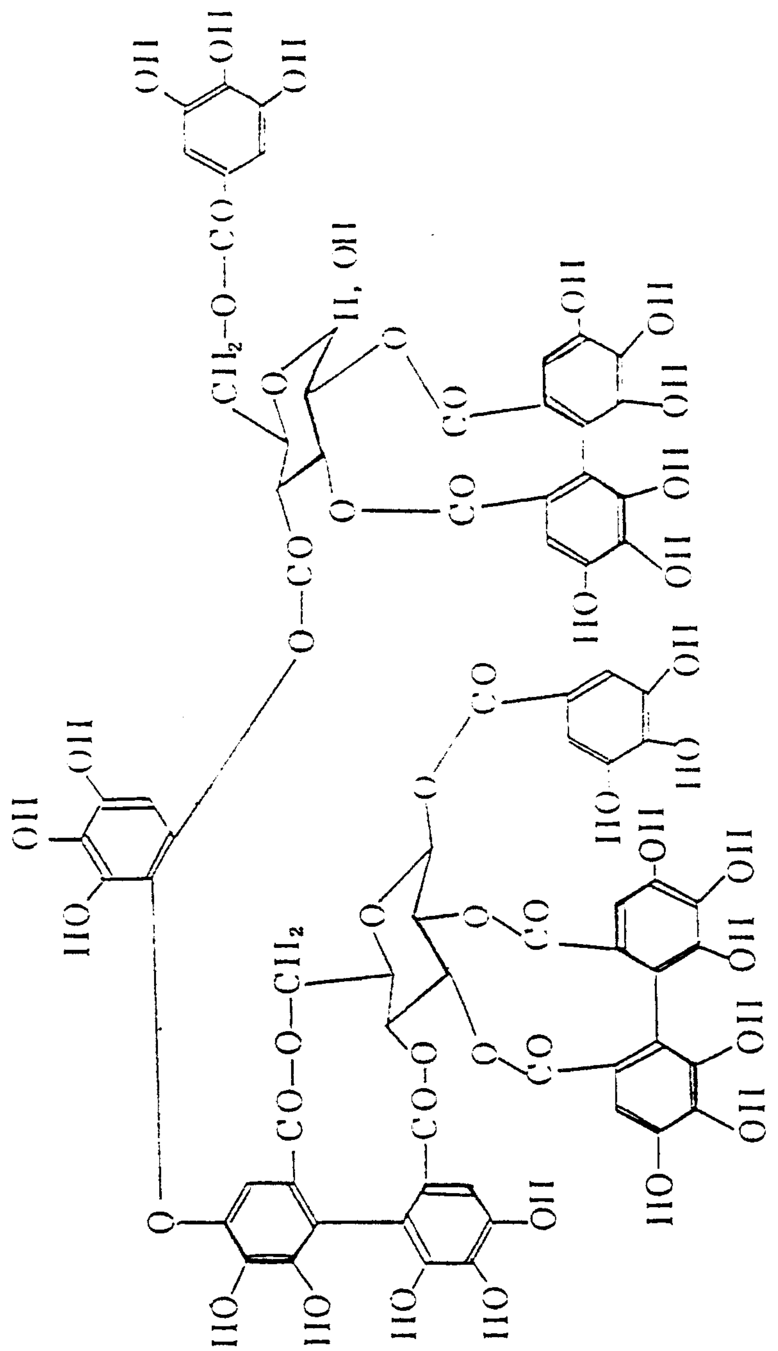

Color. Appearance:
Greish white amorphous powder
Specific Rotary Power:
$[\alpha]_D$ + 51° (c = 1.0, MeOH)
Elementary Analysis:
Molecular Formula $C_{75}H_{52}O_{48} \bullet 8H_2O$
Theoretical (%) C, 48.42 ; H, 3.54
Measured (%) C, 48.25 : H, 3.80
FAB Mass Spectrum: Parent peak not detected.
UV Spectrum :
$\lambda^{MeOH}_{max}$ nm (log $\epsilon$) :
219 (5.07), 270 (4.77)
CD Spectrum :
(MeOH), $[\theta]$ (nm) :

+ 242.000 (227). + 197,000 (235),
- 71.000 (262), + 34,000 (282),
- 19,000 (303)

Thin-layer Chromatography :
(cellulose, 7 % Acetic Acid):
Rf 0.47 ($FeCl_3$, Greyish Blue)

$^1$H-NMR Spectrum :
$\delta$ ppm (400 MHz, Acetone-$d_6$):
7.19, 7.16, 6.99, 6.92,
6.75, 6.65, 6.53, 6.49,
6.47, 6.45, 6.44, 6.43,
6.41, 6.33, 6.32, 6.22

(1) Nobotanin C

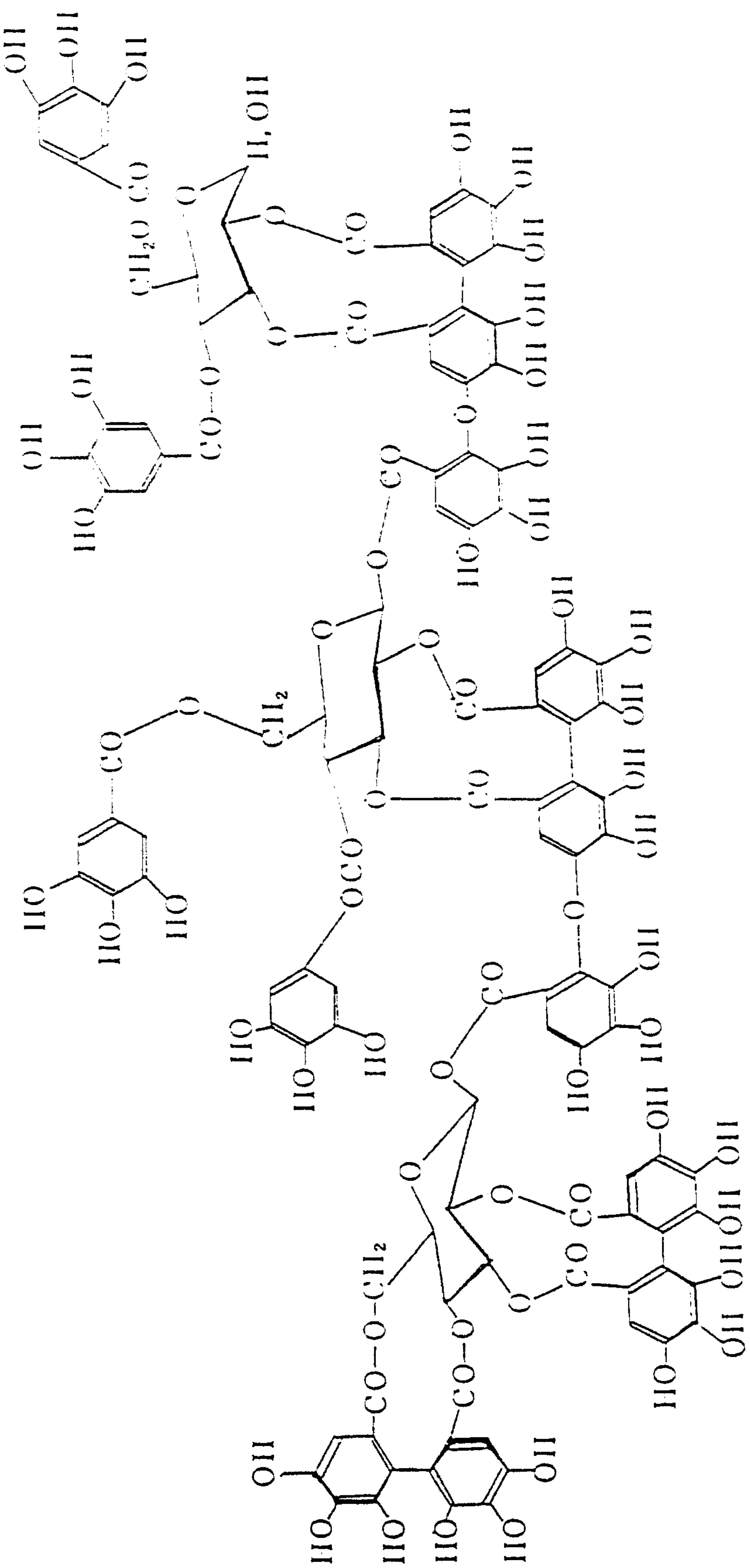

Color, Appearance:
Greyish white amorphous powder
Specific Rotary Power:
$[\alpha]_D$ + 38° (d = 1.0, MeOH)
Elementary Analysis:
Molecular Formula $C_{115}H_{80}O_{74} \bullet 22H_2O$
Theoretical (%) C, 45.62 ; H, 4.09
Measured (%) C, 45.64 ; H, 3.81
FAB-MS :
Parent peak not detected
UV :
$\lambda_{max}^{MeOH}$ nm (log $\epsilon$) :
223 (5.29), 270 (5.00)
CD :
(MeOH), $[\theta]$ (nm) :
+ 457,000 (227),
+ 356,000 (235),
- 124,000 (262),
+ 45,000 (283),
- 45,000 (312)
Thin-layer Chromatography:
(cellulose, 7 % Acetic Acid):
Rf 0.41 ($FeCl_3$ Greyish Blue)
$^1$H-NMR :
$\delta$ ppm (200 MHz, Acetone-$d_6$): 7.30, 7.28, 7.19, 7.18, 7.14, 7.13, 7.04, 7.00, 6.99, 6.71, 6.68, 6.56, 6.53, 6.51, 6.50, 6.49, 6.48, 6.45, 6.40, 6.26, 6.22, 6.16, 6.15

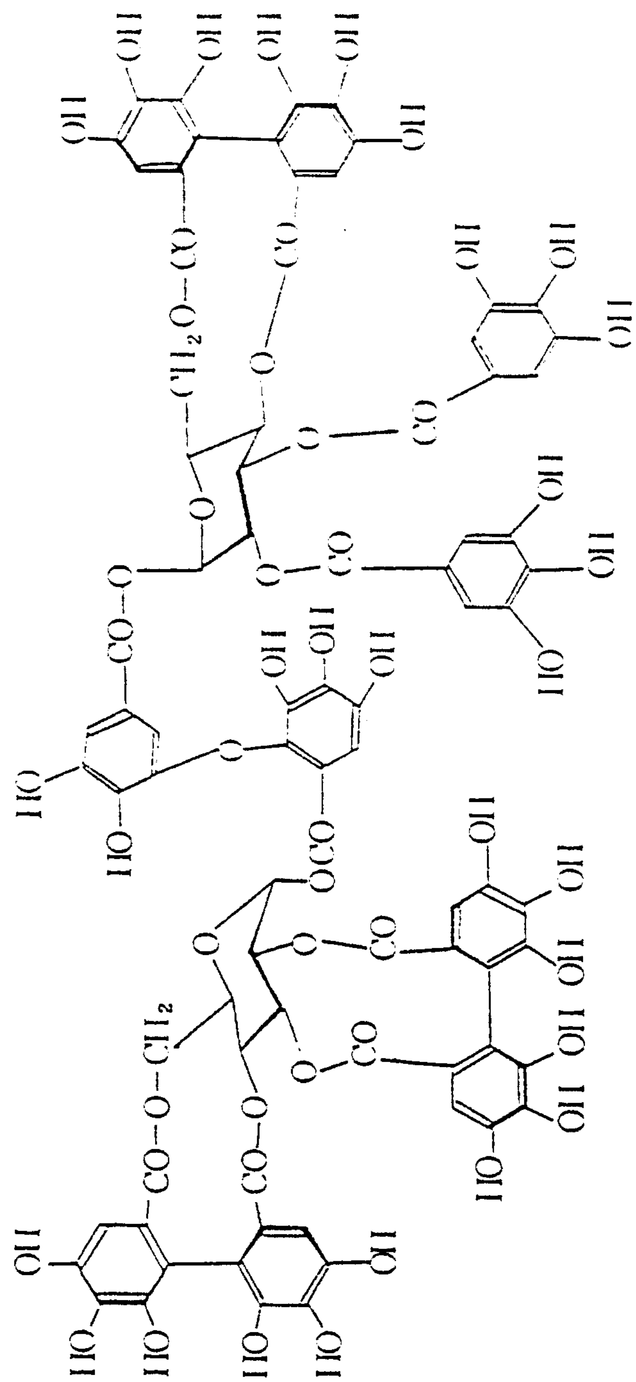

(3) Gemin A

Color, Appearance:
Greyish white amorphous powder
Specific Rotary Power:
$[\alpha]_D + 156^{\circ}$ (c = 1.6, EtOH)
Elementary Analysis:
Molecular Formula $C_{82}H_{56}O_{52} \bullet 7H_2O$
Theoretical (%) C, 49.3 ; H, 3.5
Measured (%) C, 49.2 ; H, 3.5
FAB-MS :
m/z 1,873 $[(M + H)^+]$
UV :
$\lambda_{max}^{MeOH}$ nm (log $\epsilon$) :

222 (5.26), 272 (4.92)
CD :
(MeOH), [θ] (nm) :
+ 335,000 (235),
- 68,000 (262),
+ 65,000 (283)
Thin-layer Chromatography:
(cellulose, 7 % Acetic Acid):
Rf 0.31
$^1$H-NMR :
δ ppm (200 MHz, Acetone-$d_6$): 7.35, 7.31, 7.05, 7.00, 6.88, 6.70, 6.67, 6.65, 6.56, 6.51, 6.48, 6.38, 6.17, 5.85, 5.59, 5.54, 5.38, 5.31, 5.24, 5.22, 5.19, 4.52, 3.79, 3.69

(4) Rugosin D

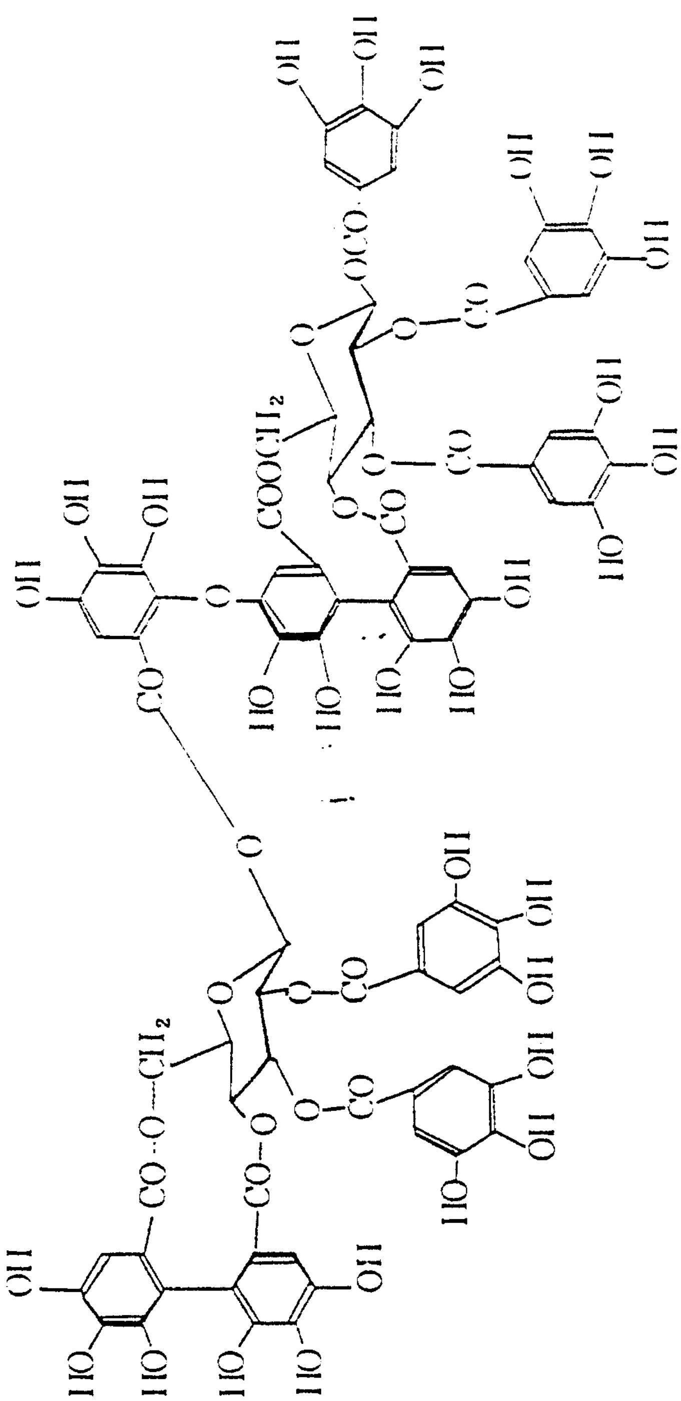

Color. Appearance:
Greyish white amorphous powder
Specific Rotary Power:
$[\alpha]_D$ + 118° (c = 1.0, Acetone)
Elementary Analysis:
Molecular Formula $C_{82}H_{53}O_{52} \bullet 9H_2O$
Theoretical (%) C, 48.34 ; H, 3.76
Measured (%) C, 48.31 ; H, 3.64
FAB MS :
m/z 1,898 [$(M+Na)^+$]
UV:

$\lambda_{max}^{MeOH}$ nm (log $\epsilon$) :
219 (5.17), 277 (4.84)

CD :
(MeOH) [$\theta$] (nm) :
+ 235,000 (227),
- 35,000 (258),
+ 78,000 (280)

Thin-layer Chromatography:
(cellulose, 7 % Acetic Acid):
Rf 0.29

$^1$H-NMR :
$\delta$ ppm (200 MHz, Acetone-$d_6$): 7.149, 7.032, 7.025, 7.02, 6.99, 7.146, 6.67, 6.49, 6.47, 6.26, 6.20, 6.15, 5.85, 5.81, 5.61, 5.54, 5.33, 5.16, 4.54, 4.48, 3.83, 3.79

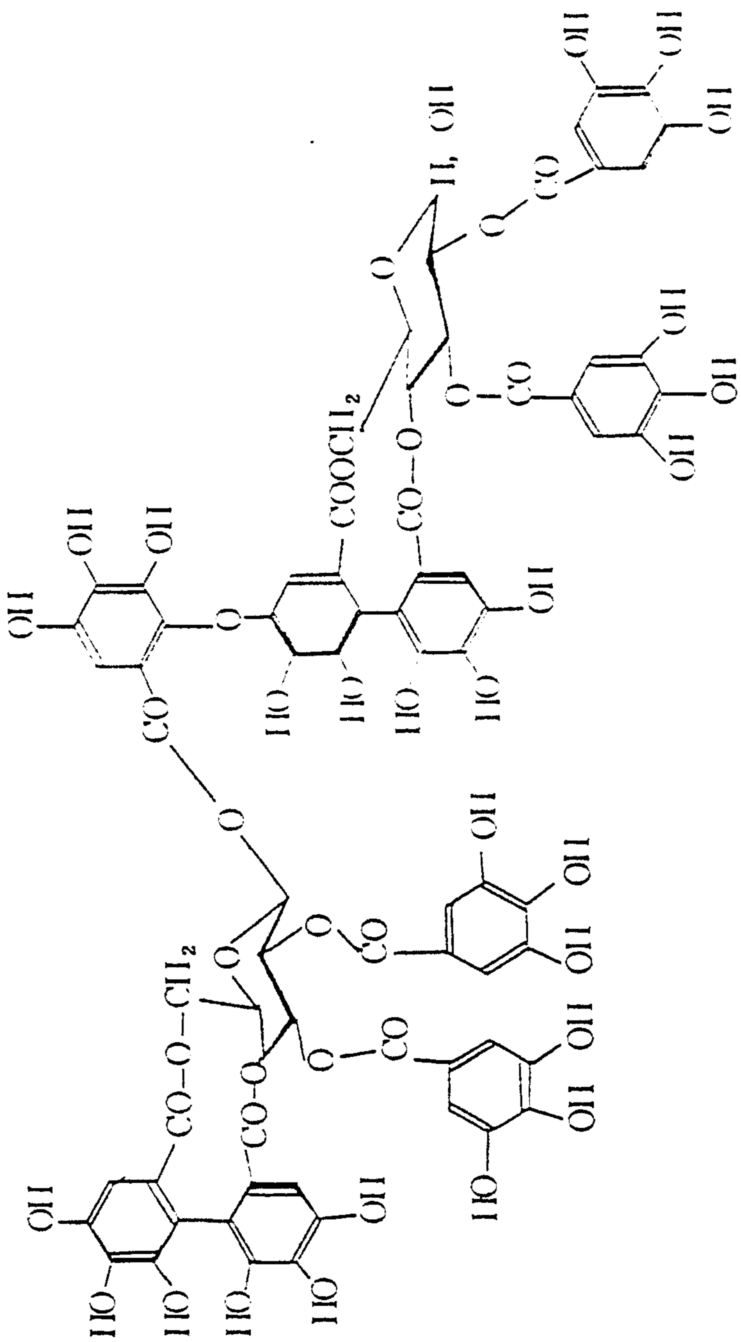

(5) Rugosin E

Color. Appearance:

Light-brown amorphous powder

Specific Rotary Power:

$[\alpha]_D$ + 140° (c = 1, Acetone)

Elementary Analysis:

Molecular Formula $C_{75}H_{54}O_{48} \bullet 5H_2O$

Theoretical (%) C, 49.58 ; H, 3.56

Measured (%) C, 49.78 ; H, 3.27

FAB-MS :

Parent peak not detected.

UV :

$\lambda_{max}^{MeOH}$ nm (log $\epsilon$) :

220 (5.19), 275 (4.87)
CD :
(MeOH) [θ] (nm) :
+224,000 (224),
- 53,000 (258),
+ 96,000 (282)
Thin-layer Chromatography:
(cellulose, 7 % Acetic Acid):
Rf 0.34
$^1$H-NMR :
δ ppm (200 MHz, Acetone-$d_6$): 7.16, 7.09, 7.08, 7.034, 7.028, 6.99, 6.67, 6.49, 6.47, 6.251, 6.245, 6.17

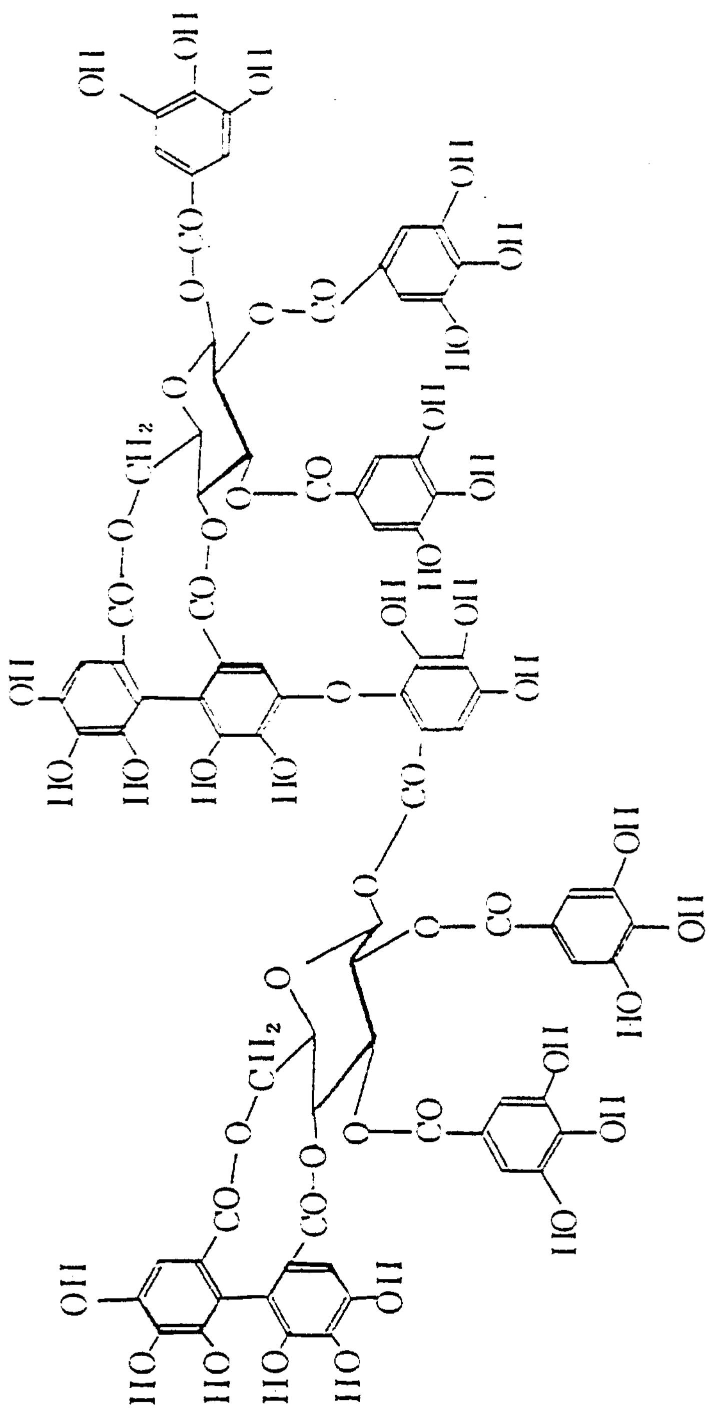

(6) Isorugosin D

Color, Appearance:
Light-brown amorphous powder.
Specific Rotary Power:
$[\alpha]_D$ + 75° (c = 0.5, MeOH)
Elementary Analysis:
Molecular Formula $C_{82}H_{58}O_{52} \bullet 7H_2O$
Theoretical (%) C, 49.20 ; H, 3.62
Measured (%) C, 49.49 : H, 3.77
FAB-MS :
m z 1,897 $[(M+Na)^+]$
UV :
$\lambda_{max}^{MeOH}$ nm (log $\epsilon$) :

218 (5.33)
277 (4.86)
CD :
(MeOH) [θ] (nm) ;
+ 31,000 (285)
- 602,200 (255)
+ 24.95 (228)
Thin-layer Chromatography:
(cellulose, 7 % Acetic Acid): Rf 0.40
$^1$H-NMR :
δ ppm (400 MHz, Acetone-$d_6$): 7.24, 7.12, 7.07, 7.01, 6.99, 6.94, 6.69, 6.65, 6.51, 6.19, 6.17, 6.12, 5.82, 5.63, 5.56, 5.54, 5.30, 5.22, 5.21, 5.10, 4.51, 4.34, 3.87, 3.86

(7) Cornusiin A

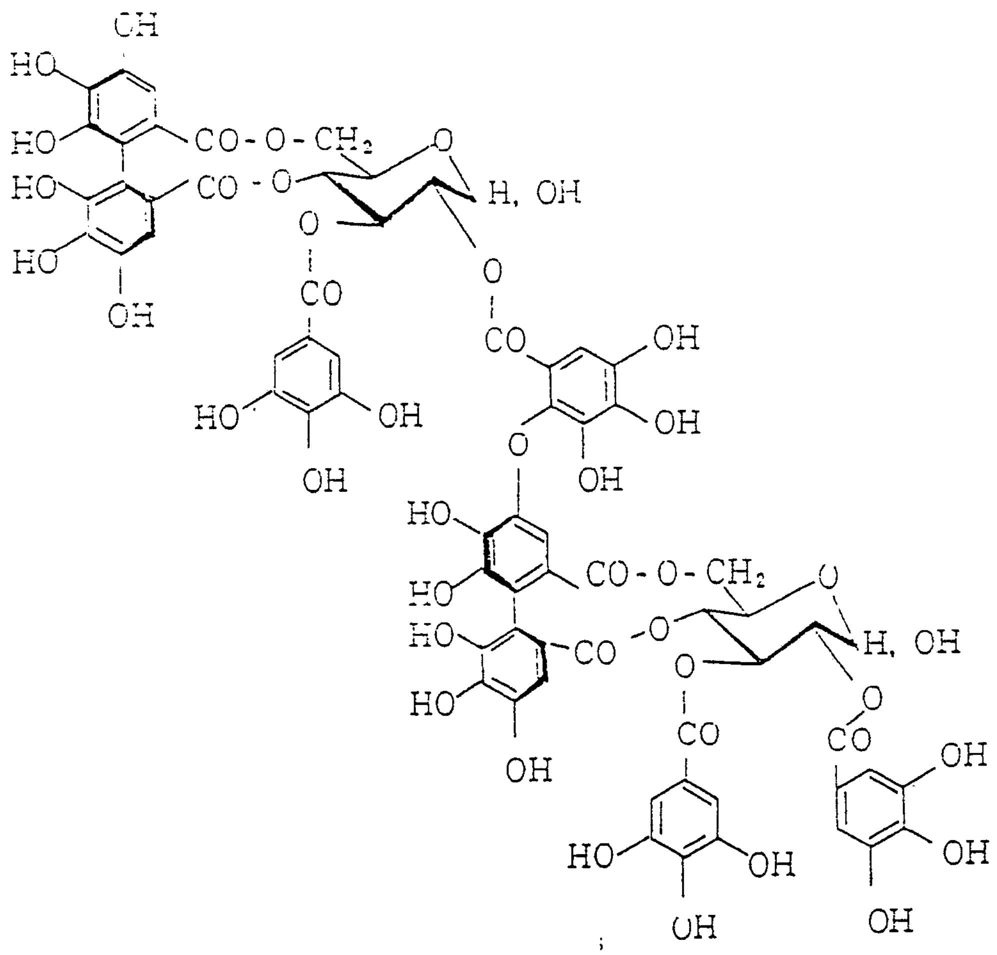

Color, Appearance:
Greyish white amorphous powder
Specific Rotary Power:

$[\alpha]_D$ + 78° (c = 1.0. MeOH)
Elementary Analysis:
Molecular Formula $C_{68}H_{50}O_{44} \bullet 11H_2O$
Theoretical (%) C. 46.16 : H. 4.10
Measured (%) C. 46.21 : H. 3.97
FAB-MS :
m z 1.593 [(M+Na)$^+$]
UV :
$\lambda_{max}^{MeOH}$ nm (log $\epsilon$) :
218 (5.14).
271 (4.81)
CD :
(MeOH) [$\theta$] (nm) :
+ 330,000 (220).
+ 200.000 (230).
- 130.000 (260).
+ 130.000 (285)
Thin-layer Chromatography:
(cellulose. 7 % Acetic acid):
Rf 0.34
$^1$H-NMR :
$\delta$ ppm (400 MHz. Acetone - $d_6$):
Aromatic Proton
7.05 - 7.04 (2 Hin total).
7.01 - 6.97 (2H). 6.90 - 6.81 (2H).
7.09 - 7.04 (1H. partly overlapping with signal of galloyl group). 6.71 - 6.67 (1H).
6.65 - 6.63 (1H). 6.49 - 6.46 (1H).
6.21 - 6.16 (1H).

Examples of Peaks as Differentiated by Formation of Anomer Mixture:

One Aromatic Proton:

6.21 (3 14 H),
6.18 (3 14 H),
6.17 (6 14 H),
6.16 (2 14 H),

Aromatic Proton of One Galloyl group:

6.90 (6 14 H).
6.87 (4 14 H).
6.85 (12 14 H),
6.81 (6 14 H)

3-Positioned Proton of α-Anomer of Both Glucose Cores:

5.83 (3/14 H),
5.81 (2/14 H),
5.72 (3/14 H),
5.70 (6/14 H)

1-Positioned Proton of β-Anomer of Left-side Glucose:

4.61 (6/14 H),
4.53 (3/14 H)

(Because the compound includes four kinds of anomers, this showed the complicated NMR spectrum as above.)

(8) Coriariin A

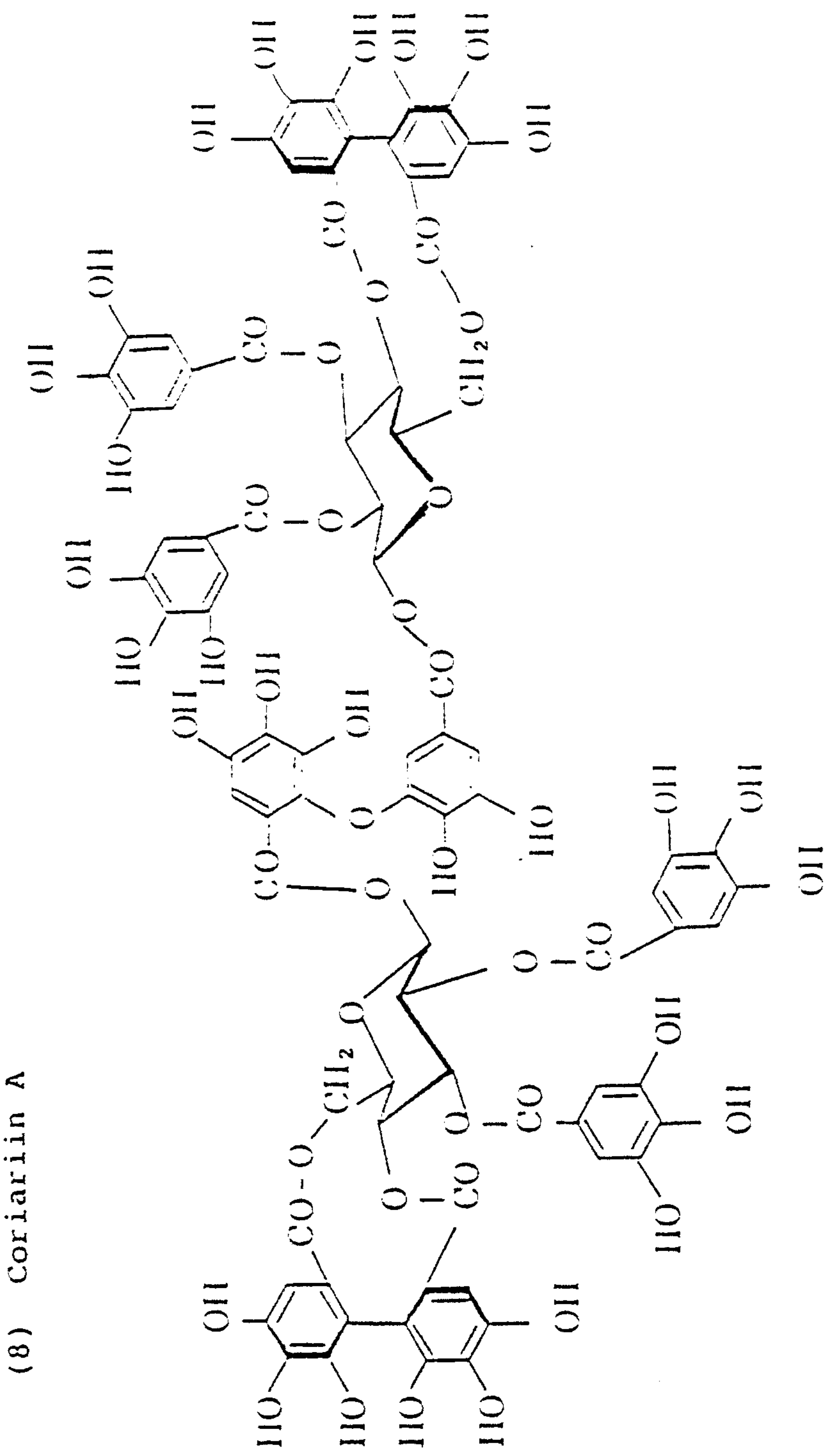

Color. Appearance:

Greyish white amorphous powder.

Specific Rotary Power:

$[\alpha]_D$ + 91° (c = 0.1. Acetone)

Elementary Analysis:

Molecular Formula $C_{82}H_{58}O_{52} \bullet 12H_2O$

Theoretical (%) C. 47.09 : H. 3.95

Measured (%) C. 47.02 : H. 4.08

FAB-MS :

m/z 1.898 [ $(M+Na)^+$ ]

UV :

$\lambda_{max}^{MeOH}$ nm (log $\epsilon$) :
224 (5.20),
280 (4.92)
CD :
(MeOH) ($\theta$] (nm) :
+ 284.000 (230),
- 38,000 (261),
+ 60,000 (282)
Thin-layer Chromatography:
(cellulose, 7% Acetic Acid): Rf 0.27
$^1$H-NMR :
$\delta$ ppm (200 MHz, Acetone-$d_6$): 7.27, 7.18, 6.70, 7.039, 7.036, 6.991, 6.985, 6.68, 6.65, 6.51, 6.12, 6.06, 5.83, 5.81, 5.61, 5.59, 5.37, 5.35, 5.26, 5.21, 4.51, 4.47
(In addition, H-6′ signal of glucose overlaps with the peak of HDO at near 3.8 ppm.)

(9) Coriariin C

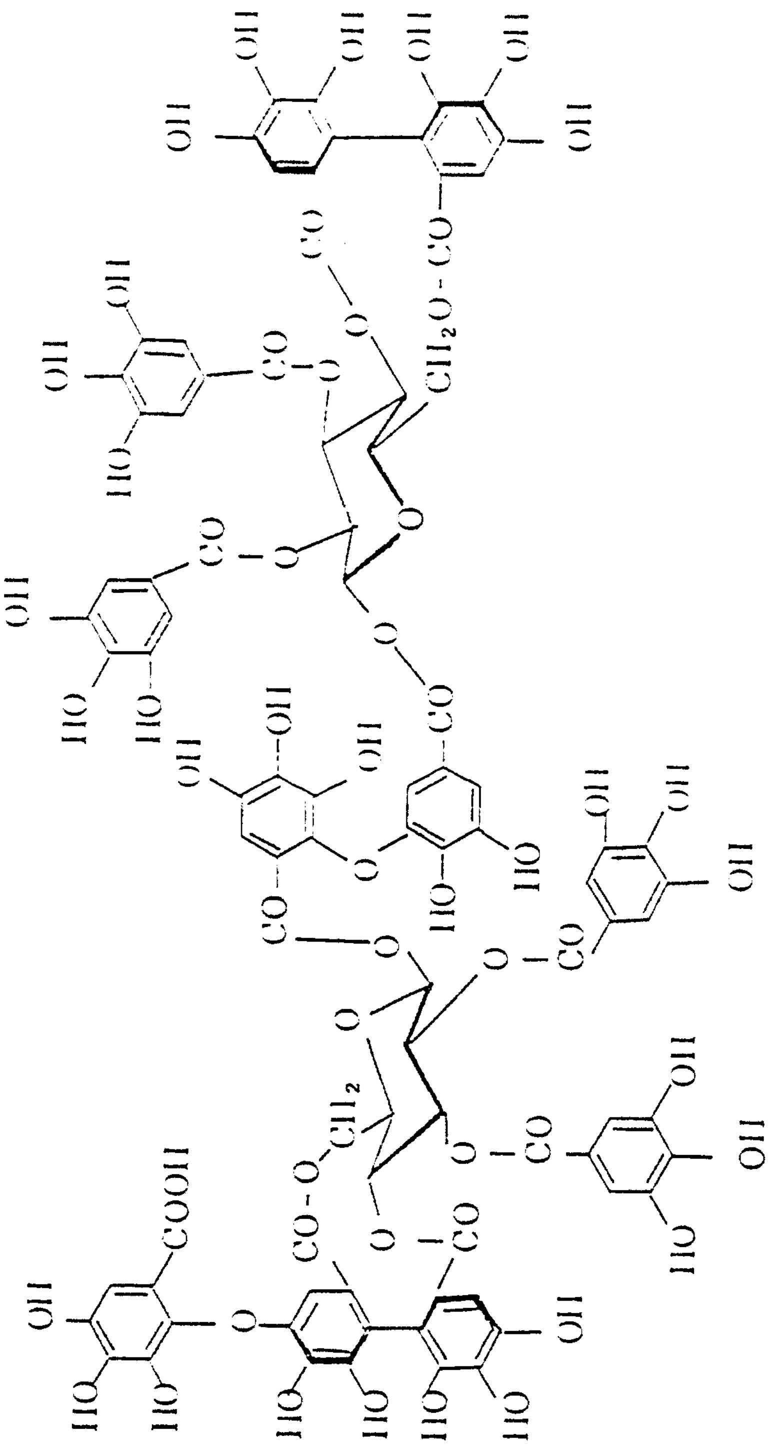

Color. Appearance:
Light brown amorphous powder.
Specific Rotary Power:
$[\alpha]_D$ + 99° (c = 0.1. MeOH)
Elementary Analysis:
Molecular Formula $C_{89}H_{52}O_{57} \bullet 14H_2O$
Theoretical (%) C, 46.57 ; H, 3.95
Measured (%) C, 46.63 ; H, 4.15
FAB-MS :
m.z 2,065 [$(M+Na)^+$]
UV :

λ $^{MeOH}_{max}$ nm (log ε) :
220 (5.24),
277 (4.93)
CD :
(MeOH) [θ] (nm) ;
+ 350,000 (231),
- 50,000 (263),
+ 69,000 (285)
Thin-layer Chromatography:
(cellulose, Upper Layer of n-BuOH-n-PrOH-$H_2O$ (2:1:3 v/v))
Rf 0.43
$^1$H-NMR : δ ppm (200 MHz, Acetone-$d_6$); 7.23
7.17, 6.68, 7.006, 6.993, 6.968, 6.965,
7.15, 6.66, 6.46, 6.45, 6.36, 6.10, 6.01,
5.55, 5.51, 5.82, 5.76, 5.19, 5.10, 4.49,
4.42, 5.33, 5.25, 3.84, 3.69

(10) Oenothein B

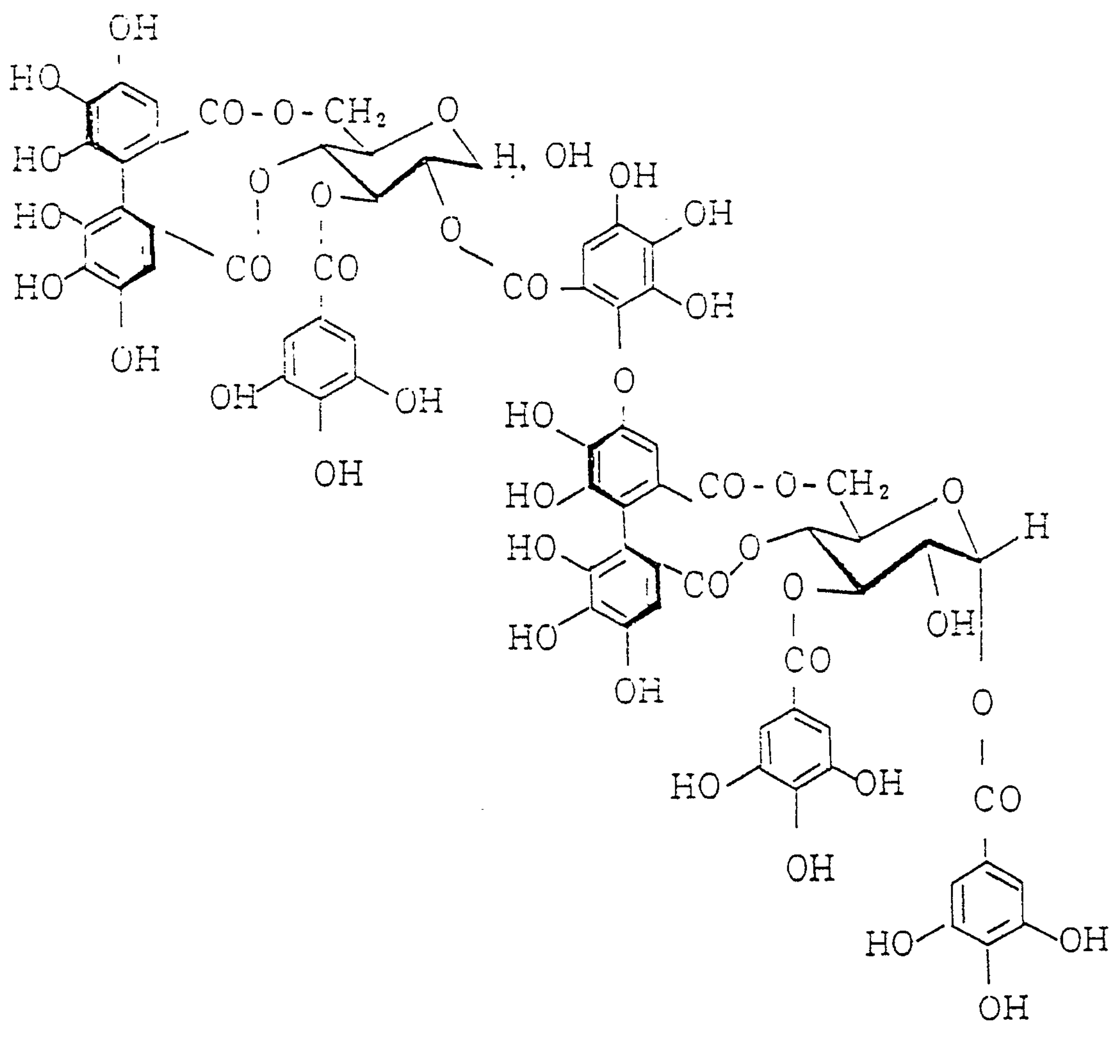

Color, Appearance:

Greyish white amorphous powder
Specific Rotary Power:
$[\alpha]_D$ + 220° (c = 0.5. MeOH)
Elementary Analysis:
Molecular Formula $C_{58}H_{50}O_{44}\cdot 8H_2O$
Theoretical (%) C. 47.62 ; H. 3.88
Measured (%) C, 47.47 : H. 3.61
FAB-MS :
m z 1.593 $[(M+Na)^+]$
UV :
$\lambda_{max}^{MeOH}$ nm (log ε) :
218 (5.08).
268 (4.77)
DC : (MeOH) [θ] (nm) :
+ 379.000 (218).
- 43.200 (258).
+ 104.000 (280)
Thin-layer Chromatography:
(cellulose.7 % Acetic Acid): Rf 0.50
$^1$H-NMR :
δ ppm (200 MHz Acetone - $d_6$): 7.25. 7.19. 7.00. 6.70. 6.66. 6.50. 6.31, 6.20. 6.11. 5.87. 5.60. 5.45. 5.25, 5.19. 5.00. 4.92. 4.59. 4.48. 4.16, 3.85

(11) Agrimoniin

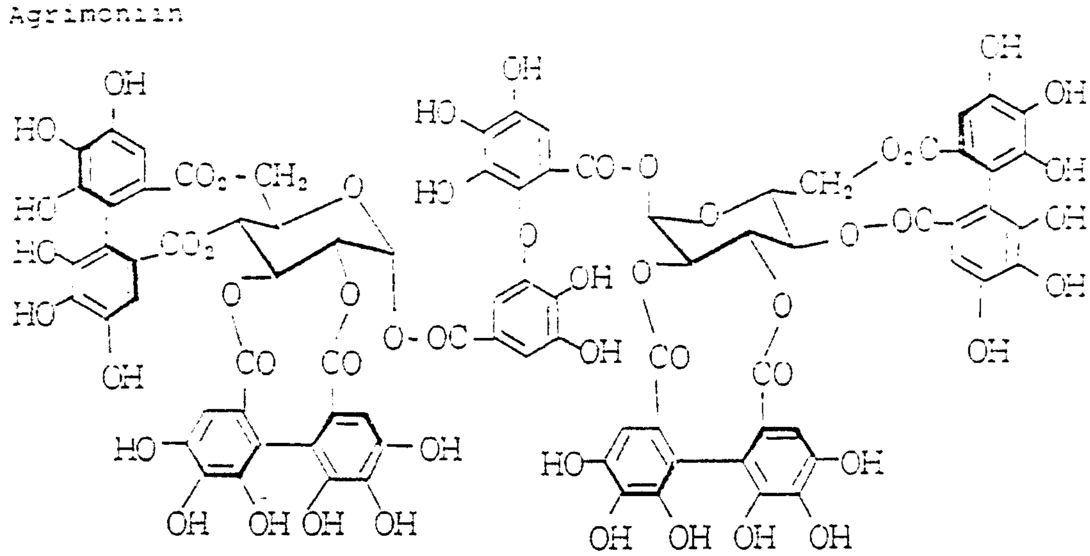

Color. Appearance:
Brown powder
Specific Rotary Power:
$[\alpha]_D^{24}$ + 140° (c = 1.94. EtOH)
Elementary Analysis:
Molecular Formula $C_{82}H_{54}O_{52}\cdot 3H_2O$
Theoretical (%) C. 51.16 ; H, 3.14
Measured (%) C. 51.29 : H. 3.17
FAB Mass Spectrum:
m z 1893 $[(M+NA)^+]$

IR Spectrum $\nu_{max}^{KBr}$ cm⁻¹:
3392 (OH), 1738 (c=O), 1620, 1516, 1446 (aromatic)
UV Spectrum $\lambda_{max}^{MeOH}$ nm (log ε):
229 (5.20), 270 (shoulder, 4.90)
¹H-NMR Spectrum:
δ ppm (200 MHz, Acetone-$d_6$) : 7.39 (1H, d, J = 2 Hz), 7.31 (1H, s), 6.94 (1H, d, J = 2 Hz), 6.663 (1H, s), 6.655 (1H, s), 6.614 (1H, s), 6.607 (1H, s), 6.57 (1 H, d, J = 4 Hz), 6.56 (1 H, s), 6.56 (1 H, d, J = 4 Hz), 6.44 (1 H, s), 6.36 (1 H, s), 6.35 (1 H, s), 5.61 (1 H, t, J = 10 Hz), 5.50 (1 H, t, J = 10 Hz), 5.37 (1 H, dd, J = 10 4 Hz), 5.36 (1 H, dd, J = 10:4 Hz), 5.25 (1 H, t, J = 10 Hz), 5.17 (1 H, t, J = 10 Hz), 5.11 - 5.27 (2 H, m), 4.64 (1 H, dd, J = 10 6 Hz), 4.49 (1 H, dd, J = 10/6 Hz), 3.79 (1 H, d, J = 14 Hz), 3.70 (1 H, d, J = 14 Hz)
¹³C-NMR Spectrum:
δ ppm (200 MHz, Methanol - $d_4$): 170.6 (s), 170.3 (s), 169.8 (s), 169.7 (s), 169.5 (s), 169.1 (s), 168.9 (s), 165.6 (s), 165.4 (s), 148.3 (s), 147.3 (s), 145.8 (s), 144.9 (s), 144.7 (s), 144.2 (s), 142.3 (s), 141.1 (s), 140.3 (s), 137.6 (s), 137.4 (s), 137.2 (s), 126.7 (s), 126.4 (s), 126.3 (s), 126.1 (s0, 125.8 (s), 125.5 (s), 120.1 (s), 116.8 (s), 116.5 (s), 116.3 (s), 115.9 (s), 115.54 (s), 115.45 (s), 115.1 (s), 114.9 (s), 112.7 (d), 110.4 (d), 108.9 (d0, 108.7 (d), 108.3 (d), 107.9 (d), 91.9 (d), 91.4 (d), 76.6 (d), 76.4 (d), 75.0 (d), 71.5 (d), 71.4 (d), 69.6 (d), 69.1 (d), 63.7 (t).

(12) Geraniin

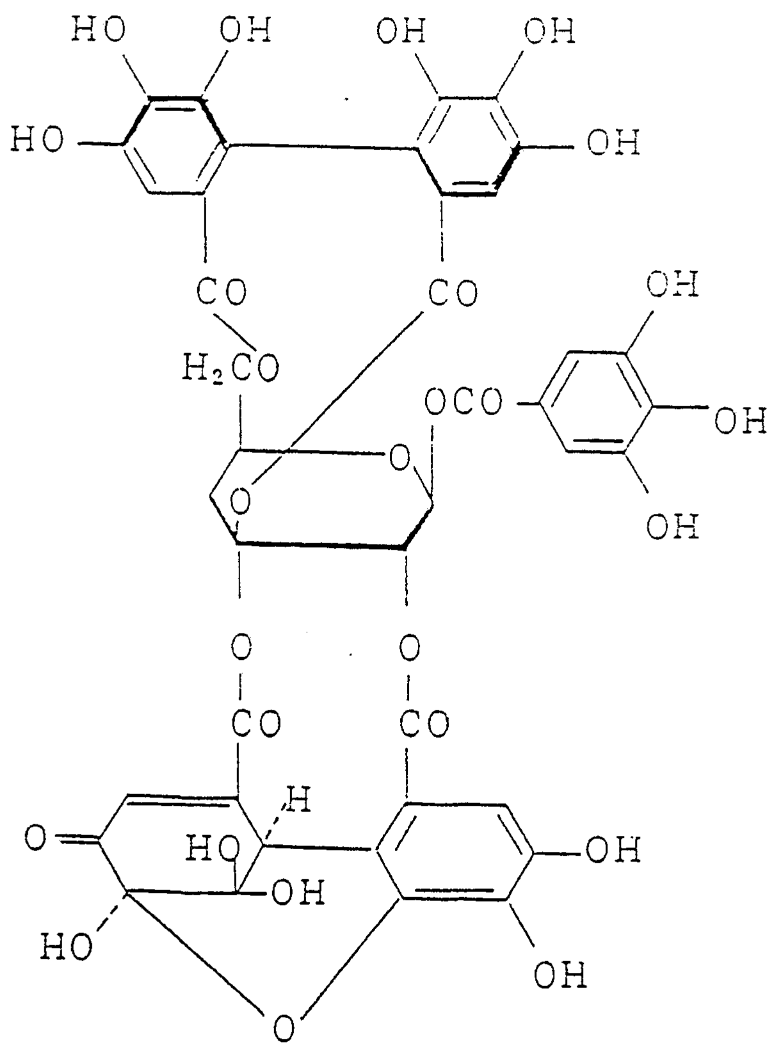

(13) Granatin A

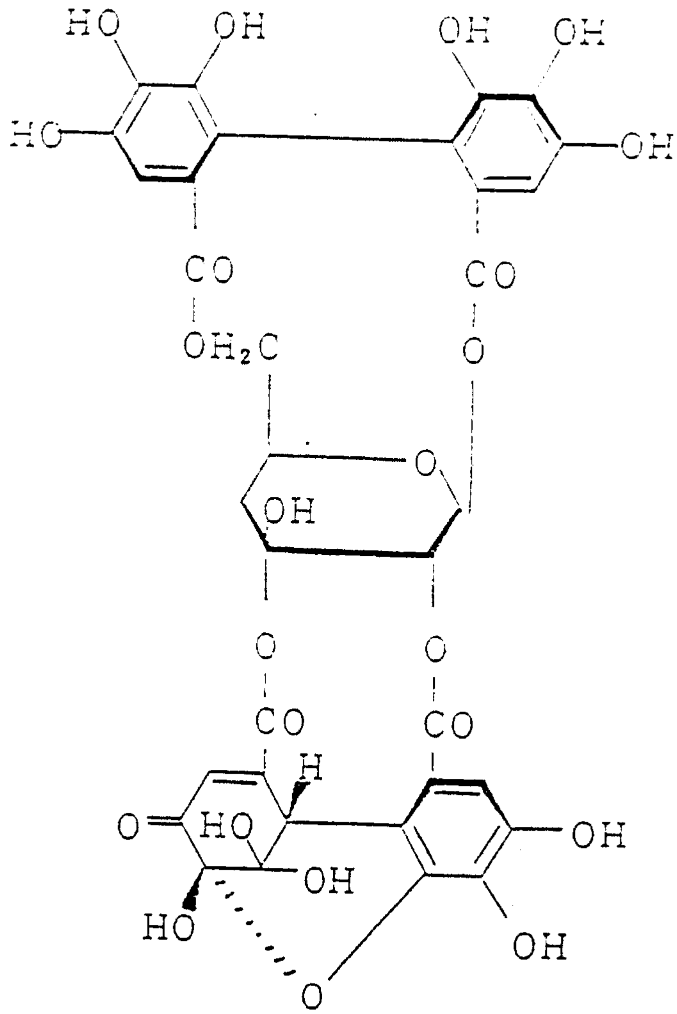

(14) Granatin B

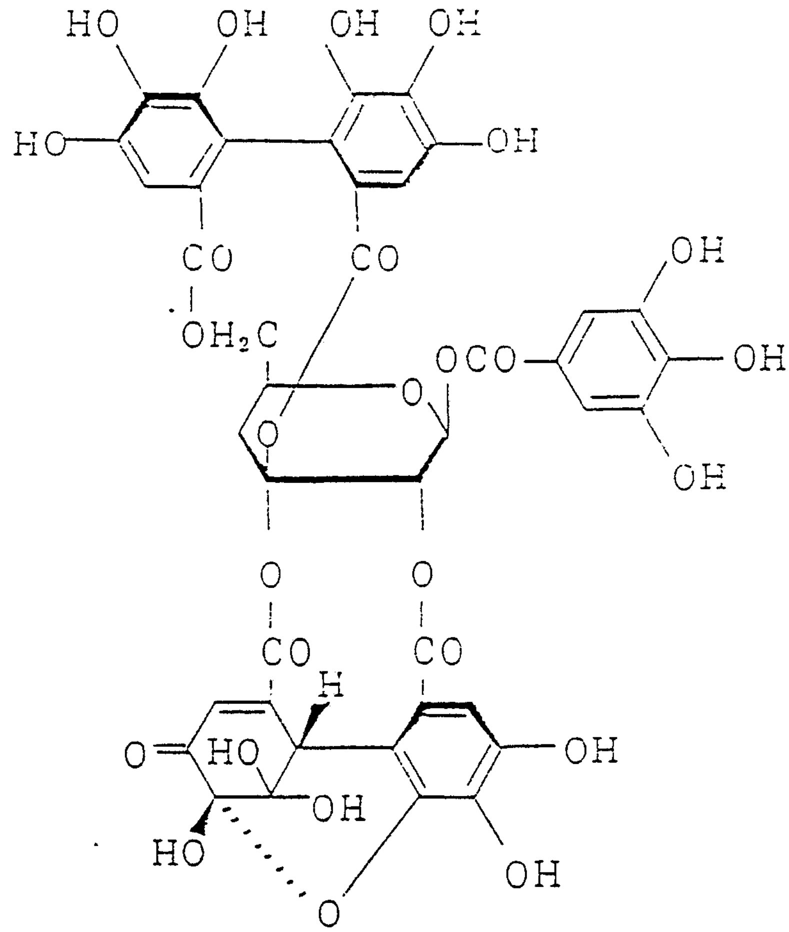

(15) Cornusiin C

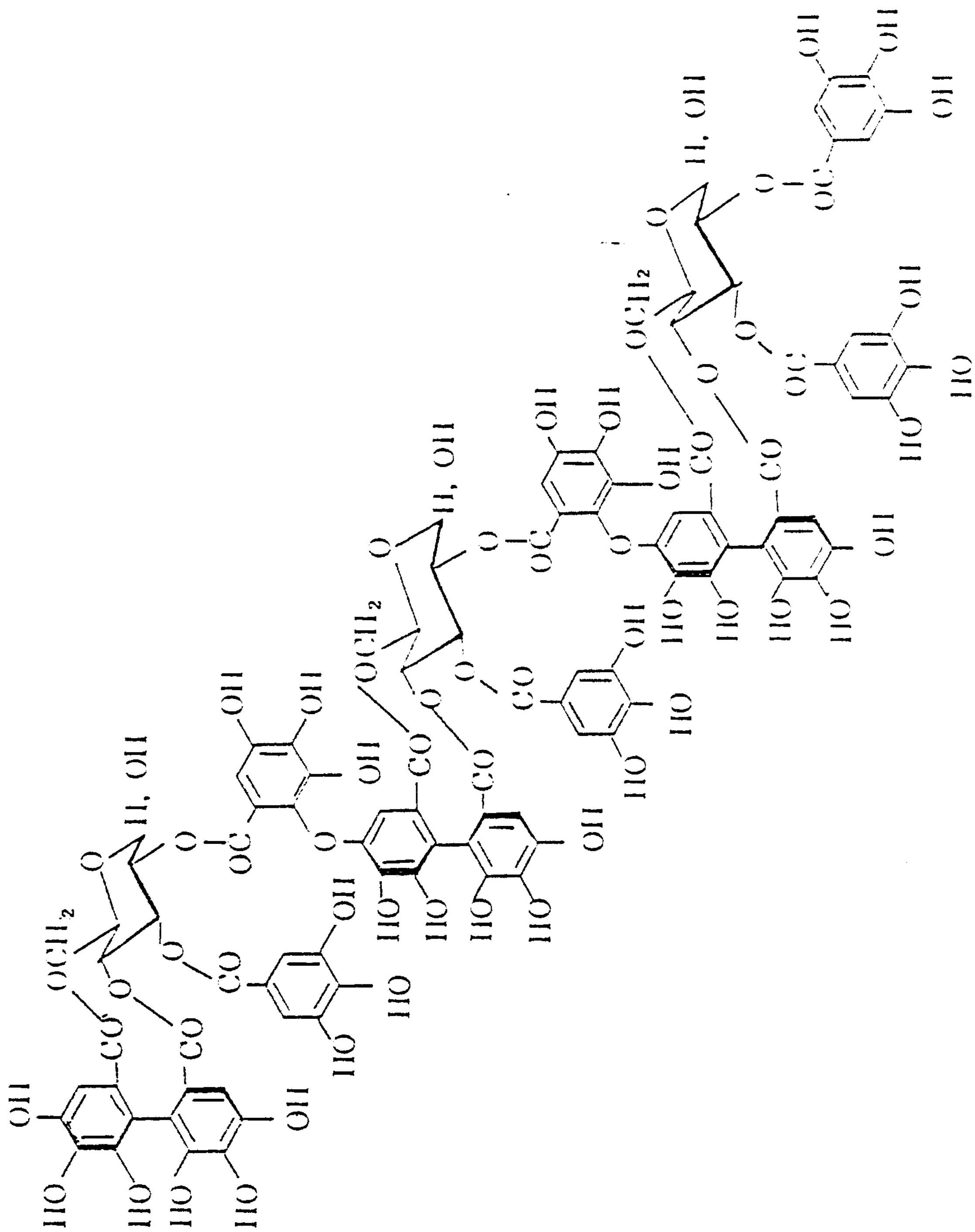

For preparation of the above-mentioned compounds, fresh or dried plant materials are homogenized in a highly polar solvent of water, methanol, ethanol, acetone or a mixture thereof and then filtered, the resulting filtrate is concentrated under reduced pressure and at 40°C or lower, low polar solvent-soluble substances are removed from the resulting concentration extract by extraction with chloroform, ether or the like, the remaining high polar solvent layer is extracted with ethyl acetate or BuOH, and the resulting extract is subjected to appropriate adsorption or partition chromatography so that the above-mentioned compounds are isolated and purified. For isolation and purification of the compounds, for example, column chromatography with a filler such as Sephadex LH20 (Pharmacia), Toyoperl HW-40 (Toyo Soda), Amberlite XAD (Organo), Diaion HP-20 (Mitsubishi Kasei) or the like, droplet countercurrent chromatography, centrifugal countercurrent chromatography or high performance liquid chromatography with Develocil 60-5 (Nomura Chemical) can be employed.

The names of the plants and the parts thereof which are used for obtaining the medicine of the present invention are mentioned in the following Table.

Table

| Tannin | Name of Plant | Parts of Plant to be used |
|---|---|---|
| Nobotanin A | Tibouchina semidecandra Cogn. | Leaves |
| Nobotanin C | Tibouchina semidecandra Cogn. | Leaves |
| Gemin A | Geum japonicum Thunberg. | Leaves |
| Rugosin D | Rosa rugosa Thunberg. | Flowers |
| Rugosin E | Rosa rugosa Thunberg. | Flowers |
| Isorugosin D | Liquidambar formosana Hance | Leaves |
| Cornusiin A | Cornus officinalis Sieb. et Zucc. | Seeds |
| Coriariin A | Coriaria japonica A. Gray | Leaves |
| Coriariin C | Coriaria japonica A. Gray | Leaves |
| Oenothein B | Oenothera erythrosepala Borbas | Leaves |
| Agrimoniin | Agrimonia Pilosa Ledeb | Roots |

Regarding the tannins' action of inactivating HIV in vitro, the reaction between the phenolic hydroxyl group and the protein of the virus is first considered to result in inactivation of the virus. However, in the tannins of the present invention, since the activity does not lower by treatment with albumin, the inactivation could not be considered to result from the simple reaction with the protein of the virus but the tannins are considered to interfere with the propagation of HIV in cells. For the interference in the propagation of HIV, there may be mentioned, for example, reverse transcriptase inhibition, inhibition of recombination of DNA into T cell DNA, inhibition of synthesis of RNA, inhibition of synthesis of virus protein, inhibition of germination, enhancement of production of interferons by cells, etc.

In the incubation period before the manifestation of the symptoms of AIDS, if the tannins of the present invention are administered to the AIDS carriers, the manifestation of the symptoms of the disease could be prevented in the carriers. in addition, even after the manifestation of the symptoms of AIDS, if the tannins of the present invention are administered to the AIDS patients, it is expected that the propagation of HIV could be inhibited so that the AIDS symptoms could be reduced because of the immunoactivating action and the antiviral action.

The active ingredients for the medical composition of the present invention for remedy of acquired immune deficiency syndrome (AIDS) and for prevention of manifestation of the symptoms of the disease can be formed into medical preparations of various types in a conventional manner, for example. in the form of liquid preparations, powders, granules, tablets, enteric coated pills, capsules or the like, by the use of conventional solvents, vehicles, auxiliary agents and the like which are generally used in formation of medical preparations.

In addition, the medical composition of the present invention for remedy of acquired immune deficiency syndrome (AIDS) and for prevention of manifestation of the symptoms of the disease may additionally contain any other active ingredients.

For peroral administration, the active ingredients of the present invention can be prescribed together with at least one vehicle, for example, starch, lactose, sucrose, mannitol, carboxymethyl cellulose or the like, into tablets, pills, capsules, powders, granules or the like.

The preparations of the kinds may further contain a lubricant such as magnesium stearate, sodium laurylsulfate, talc, etc.; a binder such as dextrin, crystalline cellulose, gum arabic, corn starch, gelatin, etc.: and a disintegrator such as potato starch, carboxymethyl cellulose, etc., in addition to the above-mentioned vehicle. In addition, the medicine of the present invention can be administered in the form of a suspension, an emulsion, a syrup or an elixir, and these preparations may contain a flavour, a coloring agent or the like, if desired.

For preparation of an injection containing the active ingredient of the present invention, in general, a distilled water for injection, a physiological salt solution, an aqueous dextrose solution, a vegetable oil for injection, propylene glycol, polyethylene glycol or the like can be used as a diluent. In addition, an isotonic agent, a stabilizer, an antiseptic, a pain depressing agent or the like may also be added to the injection, if

desired. In the case of the preparation of this kind. the preparation is preferably dissolved in a sterilized injection medium.

Description of the Preferred Embodiment:

The present invention will be explained in greater detail with reference to the following examples. which. however. are not intended to restrict the scope of the present invention.

Manufacture Example 1:

600 g of a dried powder of leaves of Coriaria japonica A. Gray was homogenized three times with 6 liters of an aqueous acetone solution (acetone water = 7 3. v v) and filtered, and the resulting filtrate was concentrated under reduced pressure at 40 °C. The thus concentrated extract was further extracted six times with 600 ml of chloroform to remove the chloroform-soluble fraction therefrom. The remaining aqueous layer was extracted six times with 600 ml of ethyl acetate. The organic layers were combined and the solvent was evaporated out therefrom to obtain 73.5 g of an ethyl acetate extract. The remaining aqueous layer was extracted seven times with 600 ml of n-butanol, and the organic layers were combined. The solvent was evaporated out therefrom and 55.8 g of an n-butanol extract was obtained. 2.2 g of the ethyl acetate extract and 3.2 g of the n-butanol extract were subjected to column chromatography with Sephadex LH-20 (diameter 22mm. length 43 cm) and Toyoperl HW-40 (diameter 22 mm. length 43 cm). respectively. For elution of the ethyl acetate extract with Sephadex LH-20. ethanol. ethanol methanol mixed solution and methanol were used as an eluent with gradually increasing the proportion of methanol in the eluent. and fractions were collected each in an amount of 5 ml. Thus fractions 873 to 877 as eluted with 100 % methanol were collected and these were dried to solid under reduced pressure to obtain 33 mg of Coriariin A which is one active ingredient of the present invention. On the other hand, for elution of the n-butanol extract with Toyoperl HV-40. the extract was first eluted with 950 ml of an aqueous ethanol solution (ethanol water = 7 3. v v) and then with ethanol acetone water = 65/5/30 (v/v), and fractions were collected each in an amount of 5 ml. The fractions 361 to 439 were collected and these were dried to solid under reduced pressure to obtain 313 mg of Coriariin A. Accoridngly, 346 mg of Coriariin A was obtained in all. and the yield thereof is about 1.4 % by weight to the weight of the dry powder of the plant material used.

Manufacture Example 2:

6.5 kg of a dried powder of leaves of Oenothera erythrosepala Borbas was homogenized three times with 65 liter of an aqueous acetone solution (acetone water = 7/3. v v) and filtered, and the resulting filtrate was concentrated under reduced pressure at 40 °C or lower to obtain 940 ml of a concentrated liquid. This was extracted ten times with 940 ml of ether to remove the ether-soluble fragments therefrom. The remaining aqueous layer was extracted five times with 400 ml of n-butanol and then the solvent was evaporated out by distillation. Thus 32 g of an n-butanol extract was obtained. 7 g of the n-butanol extract was subjected to column chromatography with Sephadex LH-20 (diameter 22 mm. length 43 cm. whereupon ethanol. ethanol methanol mixed solution and then methanol were used as an eluent with gradually increasing the proportion of methanol in the eluent. By the elution. fractions 350 to 425 were collected. each in an amount of 5 ml. and these were dried to solid under reduced pressure. Thus 538 mg of Oenothein B which is one active ingredient of the present invention was obtained. The yield thereof is 0.4 % by weight per the weight of the dry powder of the plant material used.

The substances obtained by the above-mentioned Manufacture Examples may be purified by centrifugal countercurrent chromatography or partition high performance liquid chromatography with Develosil 60-5 or the like so as to further elevate the purity of the substances. The thus purified tannins can be used as a medical composition for remedy of HIV-infections diseases, and the composition may be administered by various administration routes, for example, peroral (including buccal tablet and sublingal tablet), perrectal, pernasal, peranal, parenteral (including subcutaneous, intramuscular, intravenous, intraarterial and intracutaneous administration) or the like administration routes.

Prescription Example 1:

60 g of the active ingredient of the present invention was made into a fine powder, and this was blended with 220 g of lactose and 20 g of magnesium stearate. Using a single-shot Stegg tablet-forming machine, the mixture was formed into slug tablets each having a diameter of 20 mm and a weight of about 2.3 g. These were pulverized with an oscillator, granulated and sieved to obtain a good granule preparation of from 20 to 50 mesh.

The granule thus prepared contained the active ingredient in an amount of 200 mg per g of the granule. This may be administered to patients in a dose of from 0.5 to 2.5 g per one administration in accordance with the condition of the patients.

Prescription Example 2:

90 g of crystalline cellulose and 10 g of magnesium stearate were added to 100 g of the active ingredient of the present invention, and the resulting mixture was formed into tablets each having a diameter of 9 mm and a weight of 200 mg, using a single-shot tablet-forming machine.

The tablet contained 100 mg of the active ingredient in one tablet, and from 1 to 5 tablets may be administered to patients in accordance with the condition of the patients.

Prescription Example 3:

100 g of the active ingredient of the present invention was made into a fine powder, and 100 mg of the resulting powder was filled in hard capsules each in an amount of 100 mg per one capsule. Thus capsules containing the active ingredients of the present invention were prepared.

The capsule contained 100 mg of the active ingredient in one capsule, and from 1 to 5 capsules may be administered to patients in accordance with the condition of the patients.

Prescription Example 4:

20 g of the active ingredient of the present invention was dissolved in 1 liter of a distilled water for injection which had been warmed to 60 °C, in a conventional manner for preparation of injections. The resulting solution was made isotonic with sodium chloride and then put in ampules. The injection contained the active ingredient of the present invention in an amount of 20 mg per ml of the injection. The injection may be administered to patients by subcutaneous, intraveous or intramuscular injection, in a dose of up to 25 ml a day, in accordance with the condition of the patients.

Next, the antiviral activity of the medicine of the present invention and the safety thereof will be demonstrated by way of the following experimental examples.

Experimental Example 1: Anti-HIV Activity

(1) Cells:

CCRF-CEM (infant acute lymphocytic leukemia cells) and MT-4 (adalt T cell leukemia cells) were used. The incubation of the cells was conducted in a 10 % fetal calf serum-added RPMI 1640, at 37 °C.

(2) Viruses:

LAV strain was used. LAV strain was, after propagated in CCRF-CEM, filtered through a filter (pore size: 0.45 µm) and then used.

(3) Evaluation of Anti-HIV Activity:

CCRF-CEM ($2 \times 10^5$ ml) were infected with HIV in a rate of 1 $TCKD_{50}$ cell, and then the medicine to be examined was added thereto in an amount of 0.1, 1, 10 or 100 µg ml. At the time when the cell-degenerated effect (CDE) appeared in the positive control not containing the medicine to be examined, the cultured supernatant was collected and filtered through a filter (pore size: 0.45 µm). Thus the infected value was measured in every test case. For evaluation of the cytotoxicity, the medicine to be examined was added to the cells not infected with HIV in the same concentration as above, and the cytotoxicity was evaluated on the basis of the killed cells, if any, at the time of the above-mentioned HIV collection.

(4) Measurement of Infected Value:

MT-4 cells were used. MT-4 cells were added to a micro-plate in an amount of $2 \times 10^4$ cell 100 µl well, and then 100 µl of viruses-containing solution as stepwise diluted to have a 1 10 concentration was added. Thus the cells were incubated at 37°C. The appearance of CDE was observed for 4th to 7th days, and $TCID_{50}$ was calculated by Reed-Muench method.

| Medicine Examined | Amount of dose | Cytotoxicity | Infected Value |
|---|---|---|---|
| Positive Control | - | - | $10^{2.5}TCID_{50/0.1}$mℓ |
| Coriariin A | 100 µg mℓ<br>10 µg mℓ<br>1 µg mℓ<br>0.1 µg mℓ | +<br>-<br>-<br>- | $<10^{0.5}TCID_{50/0.1}$mℓ<br>$<10^{0.5}TCID_{50/0.1}$mℓ<br>$<10^{0.5}TCID_{50/0.1}$mℓ<br>$<10^{0.5}TCID_{50/0.1}$mℓ |
| Oenothein B | 100 µg mℓ<br>10 µg mℓ<br>1 µg mℓ<br>0.1 µg mℓ | +<br>-<br>-<br>- | $<10^{0.5}TCID_{50/0.1}$mℓ<br>$<10^{0.5}TCID_{50/0.1}$mℓ<br>$<10^{0.5}TCID_{50/0.1}$mℓ<br>$<10^{0.5}TCID_{50/0.1}$mℓ |
| Agrimoniin | 100 µg mℓ<br>10 µg mℓ<br>1 µg mℓ<br>0.1 µg mℓ | +<br>-<br>-<br>- | $<10^{0.5}TCID_{50/0.1}$mℓ<br>$<10^{0.5}TCID_{50/0.1}$mℓ<br>$<10^{0.5}TCID_{50/0.1}$mℓ<br>$<10^{0.5}TCID_{50/0.1}$mℓ |

The tannins could completely inhibit the propagation of the viruses in a concentration of from 0.1 to 10 mg ml but could not injure the virus-infected cells.

Experimental Example 2: Cytotoxicity to Hela-S3 cells

Hela-S3 cells ($1 \times 10^4$ cells ml) were applied to an Eagle's MEM medium or a 10 % fresh calf serum-containing Eagle's MEM medium, and Agrimoniin was added thereto to sensitize the cells at 37°C for 2 hours. The concentration of the Agrimoniin added was variously varied. After sensitization, the cells were washed with Hanks' solution and then incubated in a $CO_2$ incubator containing a 10 % fresh calf serum-containing Eagle's MEM medium at 37°C for 72 hours. The number of the colonies formed in 72 hours was counted and, as a result, Agrimoniin was found to have a 50 % cell-inhibiting concentration ($IC_{50}$) to Hela-S3 cells of 7.3 µg ml in the Eagle's MEM medium. In addition, $IC_{50}$ of Agrimoniin to Hela-S3 cells in the 10 % fresh calf serum-containing Eagle's MEM medium was improved to be 57.4 µg ml. From these experiments, it is noted that ellagitannins have a relatively strong direct cytotoxic action but the cytotoxic action may be reduced by addition of proteins such as serum to the culture medium.

Experimental Example 3: $LD_{50}$

50 % lethal dose ($LD_{50}$) of Coriariin A to ddy mice was obtained by probit method. The results obtained were shown below.

| Route of Administration | $LD_{50}$ (mg/kg) | |
|---|---|---|
| | Male | Female |
| Intraabdominal | 120.2 | 119.1 |
| Intravenous | 37.5 | 36.8 |
| Peroral | 1000 or more | 1000 or more |

## Claims

1. A medical composition for remedy of acquired immune deficiency syndrome (AIDS) and for prevention of manifestation of the symptoms of the disease, which is characterized by comprising tannins containing monomers and oligomers of hydrolyzable tannins as an active ingredient.

2. A medical composition as claimed in claim 1, in which the tannins are ellagitannins.

3. A medical composition as claimed in claim 2, in which the compound of ellagitannis is at least one selected from the group consisting of Nobotanin A, Nobotanin C, Gemin A, Rugosin D, Rugosin E, Isorugosin D, Cornusiin A, Coriariin A, Coriariin C, Oenothein B, Agrimoniin, Geraniin, Granatin A, Granatin B and Cornusiin C.

4. A medical composition as claimed in anyone of claims 1 to 3, which is in the form for peroral (including buccal and sublingual), perrectal, pernasal, peranal, pervaginal, or parenteral (including subcutaneous, intramuscular, intravenous, intraarterial and intracutaneous) administration.